(19) Europäisches Patentamt
European Patent Office
Office européen des brevets

(11) **EP 4 065 964 B1**

(12) # EUROPÄISCHE PATENTSCHRIFT

(45) Veröffentlichungstag und Bekanntmachung des Hinweises auf die Patenterteilung:
**23.10.2024 Patentblatt 2024/43**

(21) Anmeldenummer: **20841653.7**

(22) Anmeldetag: **26.11.2020**

(51) Internationale Patentklassifikation (IPC):
***G01N 21/64*** *(2006.01)*

(52) Gemeinsame Patentklassifikation (CPC):
**G01N 21/6408; G01N 21/6445; G01N 21/645;**
G01N 2021/6434; G01N 2021/6484

(86) Internationale Anmeldenummer:
**PCT/DE2020/101003**

(87) Internationale Veröffentlichungsnummer:
**WO 2021/104579 (03.06.2021 Gazette 2021/22)**

(54) **VERFAHREN UND OPTRODE ZUR BESTIMMUNG DER KONZENTRATION EINES ANALYTEN IN EINER PROBENFLÜSSIGKEIT**

METHOD AND OPTRODE FOR DETERMINING THE CONCENTRATION OF AN ANALYTE IN A SAMPLE FLUID

METHODE ET OPTRODE POUR DETERMINER LA CONCENTRATION D'UN ANALYTE DANS UN LIQUIDE ÉCHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.11.2019 DE 102019132525**

(43) Veröffentlichungstag der Anmeldung:
**05.10.2022 Patentblatt 2022/40**

(73) Patentinhaber: **ICHORtec GmbH**
**52159 Roetgen (DE)**

(72) Erfinder: **FEDORYCH, Oleh**
**52062 Aachen (DE)**

(74) Vertreter: **Naeven, Ralf**
**König Naeven Schmetz**
**Patent- & Rechtsanwälte**
**Eupener Straße 2a**
**52066 Aachen (DE)**

(56) Entgegenhaltungen:
**US-A1- 2008 032 312     US-A1- 2009 227 043**
**US-A1- 2009 246 888**

- **JUAN DE TORRES ET AL: "Plasmonic Nanoantennas Enable Forbidden F?rster Dipole-Dipole Energy Transfer and Enhance the FRET Efficiency", NANO LETTERS, vol. 16, no. 10, 12 October 2016 (2016-10-12), US, pages 6222 - 6230, XP055431569, ISSN: 1530-6984, DOI: 10.1021/acs.nanolett.6b02470**

**Beschreibung**

Die Erfindung betrifft ein Verfahren zur Bestimmung der Konzentration eines Analyten in einer Probenflüssigkeit gemäß dem Oberbegriff des Anspruchs 1 sowie eine Optode gemäß dem Oberbegriff des Anspruchs 8.

Die Probenflüssigkeit kann insbesondere eine biologische Flüssigkeit, wie zum Beispiel Blut, Serum interzelluläre Flüssigkeit, zerebrospinale Flüssigkeit, Schweiß oder Urin sein.

Es sind bereits diverse Verfahren zur chemischen Analyse derartiger Flüssigkeiten bekannt, zum Beispiel Flammenspektroskopie, fotometrische Verfahren, kolorimetrische Verfahren, Lumineszenz-Verfahren und Verfahren unter Einsatz von ionenselektiven Elektroden oder ionenselektiven Optoden. Die Anwendung dieser Verfahren, zum Beispiel zur Analyse von Blutproben, erfordert die Extraktion von Serum oder Plasma mittels Zentrifuge, die Vorbereitung eines exakten Volumens der Serumprobe und ihrer Verdünnung mit einem exakten Volumen von destilliertem Wasser.

Aus der EP 2805 151 B1 ist eine Optode der eingangs genannten Art bekannt, mit der der pH-Wert oder Stoffkonzentrationen in einer Probe mittels einer zeitaufgelösten Spektroskopie-Methode bestimmt werden können. Es wird vorgeschlagen, einen pHsensitiven Farbstoff in einer Polymermatrix zu immobilisieren, die sulfoniertes Polyetheretherketon (SPEEK) umfasst. Dieses Material erlaube die Bestimmung des pH-Wertes der Probe über die Abklingzeit der Lumineszenz des Sensorfarbstoffes. Bei im vorherigen Stand der Technik verwendeten Materialien für die Polymermatrix führten die Wechselwirkungen zwischen Polymermatrix und Sensorfarbstoff zu Änderungen an den optischen Eigenschaften des Sensorfarbstoffes, welche dessen Verwendbarkeit für die pH-Bestimmung stark einschränkten. Neben dem pH-Wert können auch weitere Parameter, insbesondere Stoffkonzentrationen, bestimmt werden.

Die US 5,246,867 offenbart ein Verfahren zur Messung der Zuckerkonzentration einer Probe. Hierfür wird ein Donator-Akzeptor-Paar mit der Probe in Kontakt gebracht, wobei entweder die Moleküle des fluoreszenten Donators oder die Moleküle des Akzeptors auf einem Trägermaterial immobilisiert sind. Kommt es zu einer Verbindung eines Donators mit einem Akzeptor, resultiert daraus eine messbare Veränderung der Fluoreszenz-Lebensdauer des Donators. Die nicht immobilisierten Moleküle des Donator-Akzeptorpaares konkurrieren mit dem in der Probe vorhandenen Zucker, so dass die Fluoreszenz-Lebensdauerveränderung in Abhängigkeit von der Zuckerkonzentration eliminiert oder reduziert wird. Die Änderung der Fluoreszenz-Lebensdauer wird mit phasenmodulierter Fluorimetrie oder mit zeitaufgelöster Fluorimetrie bestimmt.

US 6,395,556 B1 offenbart ein Verfahren zur Bestimmung der Konzentration eines Analyten in einer Probenflüssigkeit, wobei in die Probenflüssigkeit fluoreszierende Sensormoleküle gegeben werden, deren Fluoreszenzeigenschaften von der Konzentration des Analyten abhängen. Sowohl die Probenflüssigkeit als auch eine fluoreszierende Referenzmoleküle aufweisende Referenzflüssigkeit werden einer Anregungsstrahlung ausgesetzt, welche sowohl die Referenzmoleküle als auch die Sensormoleküle zu Fluoreszenzstrahlung anregen. Die Fluoreszenzstrahlung der Referenzflüssigkeit wird entlang einer ersten Polarisationsachse und die Fluoreszenzstrahlung der Probenflüssigkeit wird entlang einer zweiten, zur ersten im Wesentlichen senkrechten Polarisationsachse polarisiert. Einer der polarisierten Emissionen wird soweit abgeschwächt, dass die Intensitäten auf beiden Polarisationsachsen im Wesentlichen gleich sind. Aus dem Schwächungsgrad kann auf die Analytkonzentration geschlossen werden.

Die US 2010/0105048 A1 und die US 2006/0014175 A1 offenbaren jeweils ein fluoreszenz-basiertes Detektionssystem, bei dem fluoreszierende Nanokristalle genutzt werden, deren spektrale Eigenschaften nachweisbar verändert werden, wenn sich die Größe der an die Oberfläche der Nanokristalle gebundenen funktionellen Gruppen aufgrund eines Kontakts mit einem Analyten ändert. Die Moleküle in der Probe fügen funktionelle Gruppen auf dem fluoreszierenden Nanokristall hinzu oder reduzieren ihre Größe proportional zur Aktivität und Menge der Analytmoleküle. Das Detektionssystem kann zum Nachweis von Telomerase in einer Probe verwendet werden.

Aus der DE 10 2018 204 744 A1 sind ein Verfahren und eine Optode der eingangs genannten Art bekannt. Demnach wird die Konzentration von in einem Elektrolyten enthaltenen Ionen eines Analyten mittels einer in den Elektrolyten eingetauchten und mit Licht bestrahlten ionenselektiven Membran bestimmt. Referenzmessungen sollen nicht erforderlich sein. Es wird eine konzentrationsabhängige Verschiebung der spektralen Position eines auf Lumineszenz beruhenden Absorptions-, Reflexions- oder Transmissionsspektrums einer Optode gemessen. Somit ist für dieses Verfahren der Einsatz eines kostenträchtigen Spektrometers mit einer hohen Auflösung erforderlich.

Aus der US 2009/246888 A1 ist eine Testvorrichtung bekannt, welche auf einem festen Substrat mindestens zwei Muster von verschiedenen darauf immobilisierten Molekülen aufweist, wobei mindestens eines der Muster eine Abmessung zwischen etwa 1 nm und etwa 1.000 nm aufweist und die Geometrie und Identität der Moleküle so beschaffen sind, dass die Wechselwirkung zwischen ihnen in Gegenwart eines Analyten moduliert wird. Der Energietransferzwischen den beiden Fluorophoren, der als Förster-Resonanzenergietransfer (FRET) bekannt ist, wird unterbrochen, wenn der Analyt an einem Bindemittel bindet, welches auf dem festen Substrat strukturiert ist. Die Modulation des Energietransfers durch den Analyten kann zu einer Aussage über die Konzentration des Analyten genutzt werden. Dieses Verfahren liefert einen Wert, der mit der Fluoreszenzintensitätsdichte, das heißt, mit der Intensität pro Flächeneinheit in Beziehung steht, die nur in sehr begrenzten Fällen proportional zur Konzentration des Analyten sein kann, z. B., wenn alle Moleküle des Analyten an das Bindemittel gebunden sind.

Der Erfindung liegt das technische Problem zugrunde, ein Verfahren sowie eine Optode der eingangs genannten Art zur Verfügung zu stellen, mit der auf kostengünstige Weise Referenzproben oder Referenzmessungen weitgehend vermieden werden können.

Das technische Problem wird bei einem Verfahren der eingangs genannten Art mit den kennzeichnenden Merkmalen des Anspruchs 1, bei einer Optode der eingangs genannten Art mit den kennzeichnenden Merkmalen des Anspruchs 8 gelöst. Vorteilhafte Ausführungsformen ergeben sich aus den abhängigen Ansprüchen.

Die Erfindung geht somit von einem Verfahren aus, bei dem mittels einer Strahlungsquelle Anregungsstrahlung auf eine Trägereinheit gerichtet wird, die immobilisierte Moleküle eines für den Analyten sensitiven Sensorfarbstoffes aufweist und mit der Probenflüssigkeit in Kontakt steht. Durch die Anregungsstrahlung wird eine Lumineszenzstrahlung des Sensorfarbstoffes ausgelöst, der auf einen Strahlungsdetektor gegeben wird, um daraus ein Detektorausgangssignal zu generieren. Mit Hilfe einer Auswerteroutine wird die Analytkonzentration aus dem Detektorausgangssignal ermittelt, wobei eine Abhängigkeit einer Eigenschaft der Lumineszenzstrahlung von der Wechselwirkung der Konzentration des Analyten in der Probenflüssigkeit ausgenutzt wird. Erfindungsgemäß wird nun vorgeschlagen, dass für die Ermittlung der Analytkonzentration die Abhängigkeit der untersuchten Eigenschaft der Lumineszenzstrahlung von einer indirekten Austauschwechselwirkung zwischen den Molekülen des Sensorfarbstoffes untereinander ausgenutzt wird, welche über Partikel des Analyten interagieren. Partikel des Analyten können Atome, Ionen oder Moleküle sein.

Die Austauschwechselwirkung, auch Austauschenergie genannt, ist ein quantenmechanisches Phänomen, welches auftritt, wenn identische Teilchen miteinander wechselwirken. Die Austauschwechselwirkung ist eine schwache Wechselwirkung, im vorliegenden Zusammenhang in der Größenordnung von meV (Millielektronenvolt), hat jedoch eine hohe Reichweite. Im Falle der indirekten Austauschwechselwirkung erfolgt diese über den Einfluss einer weiteren Größe, die vom Partikel des Analyten eingebracht wird, z.B. des elektrischen Feldes im Falle eines ionisierten Partikels. So interagiert ein Molekül des Sensorfarbstoffes mit einem Partikel des Analyten und dieser wiederum mit mindestens einem weiteren Molekül des Sensorfarbstoffes, wodurch es zur indirekten Austauschwechselwirkung zwischen den mindestens zwei beteiligten Molekülen des Sensorfarbstoffes kommt.

Die Auswerteroutine wertet eine Größe aus, welche proportional zur Exponentialfunktion

$$\exp\left(\frac{R}{r - r_o}\right)$$

ist. Diese Abhängigkeit ist eine Folge der indirekten Austauschwechselwirkung, wobei R ein Maß für den Abstand zwischen den immobilisierten Molekülen des Sensorfarbstoffes und r ein Maß für den Abstand zwischen den Partikeln des Analyten, die mit den Molekülen des Sensorfarbstoffes interagieren, ist. Die indirekte Austauschwechselwirkung ergibt sich dann, wenn der Abstand zwischen einem beteiligten immobilisierten Molekül des Sensorfarbstoffes und einem die Austauschwechselwirkung vermittelnden Partikel des Analyten maximal $r_0$ beträgt. $r_0$ ist der sogenannte Förster-Radius (Theodor Förster, "Experimentelle und theoretische Untersuchung des zwischenmolekularen Übergangs von Elektronenanregungsenergie", Zeitschrift für Naturforschung, 4a, S. 321-327 [1949]; T. Förster, Zwischenmolekulare Energiewanderung und Fluoreszenz, Annalen der Physik, Volume 437, Ausgabe 1-2, [1948], S. 55-75). Die indirekte Austauschwechselwirkung wird relevant, wenn sowohl $R < r_0$ als auch $r < r_0$ gilt.

Die vorgenannten Größen R und r ergeben sich aus der Konzentration der immobilisierten Moleküle des Sensorfarbstoffes beziehungsweise der gesuchten Konzentration des Analyten.

So gilt für einen geringen Massenanteil der immobilisierten Moleküle des Sensorfarbstoffes an der Gesamtmasse von Trägereinheit und Sensorfarbstoff in guter Näherung:

$$R \approx \frac{1}{(N \, M \, N_a)^{1/3}}$$

wobei N die Molalität der immobilisierten Moleküle des Sensorfarbstoffes bezogen auf die Trägereinheit, M die Dichte der Trägereinheit und $N_a$ die Avogadrozahl ($\approx 6{,}022 \, 10^{23} \, \text{mol}^{-1}$) ist.

Für N können Werte von z.B. unter 6 mmol/kg gegeben sein. Für die Bestimmung von Salz-Ionen $Na^+$ kann als Sensorfarbstoff z.B. ein Tetramethylammonium-Salz (z.B. Sodium Green™) eingesetzt werden.

R ist ein Maß für den mittleren Abstand der immobilisierten Moleküle des Sensorfarbstoffes untereinander und somit ein Parameter, der durch die Optode, nämlich durch die vorgegebene, auf die Masse der Trägereinheit bezogenen Molalität der Moleküle des Sensorfarbstoffes bekannt ist. Diese Molalität ist bei der Herstellung der Trägereinheit vor-

gebbar.

$r_0$ ist abhängig von der Art des Sensorfarbstoffes und kann z.B. experimentell oder durch numerische Kalkulation ermittelt werden und bis zu 10 nm erreichen.

Die Größe r steht für den mittleren Abstand zwischen den Partikeln des Analyten und es gilt wiederum in guter Näherung mit vernachlässigbarer Masse des Analyten in der Probenflüssigkeit:

$$r \approx \frac{1}{\left(n\,m\,N_a\right)^{1/3}},$$

wobei n die Molalität des Analyten in der Probenflüssigkeit und m die Dichte der Probenflüssigkeit ist. Die Molalität n ist ein direktes Maß für die gesuchte Konzentration des Analyten.

Das erfindungsgemäße Verfahren kann so ausgeführt werden, dass als Eigenschaft der Lumineszenzstrahlung die Abhängigkeit der Lebensdauer der Lumineszenzstrahlung von der Analytkonzentration ausgenutzt wird und die Auswerteroutine zur Ermittlung der Analytkonzentration von einer bekannten Konzentration der in der Polymermatrix immobilisierten Sensorfarbstoff-Moleküle und einer bekannten Abhängigkeit der Lebensdauer der Lumineszenzstrahlung von der Sensorfarbstoff-Molekülkonzentration sowie der Analytkonzentration ausgeht.

Dabei kann das erfindungsgemäße Verfahren so ausgeführt werden, dass für die Abhängigkeit der Lebensdauer der Lumineszenzstrahlung von der Sensorfarbstoff-Molekülkonzentration sowie der Analyt-Konzentration die Proportionalität

$$\tau \sim \exp\left(\frac{R}{r - r_o}\right)$$

herangezogen wird, wobei $\tau$ die Lebensdauer der Lumineszenzstrahlung der immobilisierten und mit dem Analyten wechselwirkenden Moleküle des Sensorfarbstoffes ist.

Die Lebensdauer der Lumineszenzstrahlung kann mittels zeitaufgelöster Messung oder mittels Phasenmodulation ermittelt werden.

Alternativ zum Weg über die Ermittlung der Lebensdauer der Lumineszenzstrahlung kann die Analytkonzentration auch über einen Polarisationsgrad der Lumineszenzstrahlung bestimmt werden. Hierfür kann das erfindungsgemäße Verfahren so ausgeführt werden, dass die Anregungsstrahlung polarisiert auf die Trägereinheit gerichtet wird und von einer durch die Anregungsstrahlung ausgelösten Lumineszenzstrahlung des Sensorfarbstoffes Intensitäten $I_{\parallel}$ und $I_{\perp}$ zweier im Wesentlichen senkrecht aufeinander stehender Polarisationsrichtungen festgestellt werden und als Eigenschaft der Lumineszenzstrahlung die Abhängigkeit des Polarisationsgrades

$$P = \left|\frac{I_{II} - I_{\perp}}{I_{II} + I_{\perp}}\right|$$

von der Konzentration des Analyten genutzt wird.

Dabei kann vorteilhaft als Abhängigkeit die Proportionalität

$$P \sim \exp\left(\frac{R}{r - r_o}\right)$$

verwendet werden.

Diese Proportionalität des Polarisationsgrades von der vorgenannten natürlichen Exponentialfunktion (e-Funktion) resultiert aus der bereits oben beschriebenen indirekten Austauschwechselwirkung von immobilisierten Molekülen des Sensorfarbstoffes über Partikel des Analyten, die für einen zusätzlichen Effekt im elektrischen Feld sorgt.

Im Folgenden werden beispielhafte Ausführungsformen des erfindungsgemäßen Verfahrens sowie der erfindungsgemäßen Optode anhand von Figuren näher dargestellt. Einige der im Folgenden aufgeführten Ausführungsbeispiele befassen sich der einfacheren sprachlichen Darstellung halber jeweils mit der Bestimmung der Konzentration eines Analyten einer einzelnen Art, z.B. Kochsalz (NaCl), mittels eines bestimmten Sensorfarbstoffes, z.B. ein Tetramethylammonium-Salz (z.B. Sodium Green™). Es ist aber denkbar, dass gleichzeitig oder nacheinander die Konzentration mehrerer Analyten festgestellt wird. Zudem kann mehr als ein Sensorfarbstoff eingesetzt werden.

Es zeigt:

Fig. 1:   vier beispielhafte Varianten einer Sensorvorrichtung,

Fig. 2:   im Diagramm eine Laserpulsleistung über die Zeit,

Fig. 3:   im Diagramm eine von einem Photonenzähler ermittelte genormte Strahlungsintensität,

Fig. 4:   im Diagramm die Abklingrate der Lumineszenz in Abhängigkeit von der Konzentration von Natrium-Ionen in einer untersuchten Probenflüssigkeit,

Fig. 5:   eine fünfte Sensorvorrichtung mit einer kontinuierlichen Lichtquelle,

Fig. 6:   im Diagramm den Polarisationsgrades der Lumineszenz-Strahlung in Abhängigkeit von der Konzentration des Analyten und

Fig. 7:   eine Multikern-Lichtleitfaser mit zehn Kernsträngen.

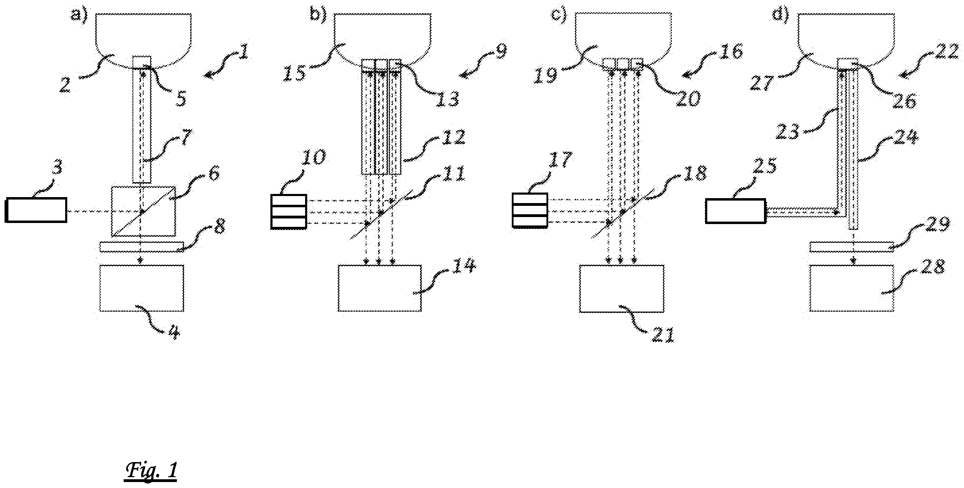

Fig. 1 zeigt vier beispielhafte Varianten einer Sensorvorrichtung, auch Optode genannt, welche für die Durchführung eines zeitaufgelösten Verfahrens zur Bestimmung der Konzentration einer chemischen Substanz (Analyt) in einer Probenflüssigkeit geeignet ist. Die Probenflüssigkeit kann in Form eines einzelnen Tropfens oder aber auch mehrerer Tropfen vorliegen. Hierbei handelt es sich bevorzugt um eine biologische Probenflüssigkeit, wie zum Beispiel Blut, Serum, zerebrospinale Flüssigkeit, interzellulären Flüssigkeit, Schweiß oder Urin.

Soweit in der folgenden Figurenbeschreibung von einem Sensorelement oder einem Sensor-Teilelement die Rede ist, weist dieses eine Trägereinheit mit immobilisierten Molekülen eines für den Analyten sensitiven Sensorfarbstoffes auf oder ist aus einer solchen Trägereinheit gebildet.

Eine erste Sensorvorrichtung 1 weist einen Probenbehälter 2, eine gepulste Lichtquelle 3, z.B. für Laserstrahlung oder LED-Strahlung, und einen Photonenzähler 4 auf. Im unteren Bereich des Probenbehälters 2, vorzugsweise innerhalb des Probenbehälters 2, befindet sich ein erstes Sensorelement 5, dessen Oberfläche in direkten Kontakt mit der zu analysierenden, hier nicht dargestellten Probenflüssigkeit kommen soll. Das Sensorelement 5 weist eine Trägereinheit aus einem funktionalisierten Polymer auf, in welchem ein für den Analyten sensitiver Sensorfarbstoff immobilisiert ist und welches bei Zufuhr der zu analysierenden Probenflüssigkeit hydriert wird. Dabei penetrieren die Partikel des Analyten das Sensorelement 5, so dass die immobilisierten Moleküle des Sensorfarbstoffes mit den Partikeln des Analyten interagieren können. Die immobilisierten Moleküle des Sensorfarbstoffes generieren eine Lumineszenz-Antwort auf das einfallende gepulste Licht, wobei Photonen der Lumineszenz-Antwort zum Photonenzähler 4 geleitet werden.

Im Falle der ersten Sensorvorrichtung 1 läuft die eingesetzte Strahlung über einen Strahlteiler 6 und eine Lichtleitfaser 7, welche z.B. eine Glasfaser sein kann. Über den Strahlteiler 6 wird das von der Lichtquelle 3 stammende Licht durch die Lichtleitfaser 7 hin zum Sensorelement 5 geführt. Von der Lumineszenz-Antwort stammende Photonen gelangen über den Strahlteiler 6 auf den Photonenzähler 4. Die von der Lumineszenz-Antwort stammenden Photonen können vor Eintritt in den Photonenzähler 4 optional einen optischen Filter 8, zum Beispiel einen Hochpassfilter, durchlaufen, der den Eintritt von Anregungsstrahlung verhindern soll.

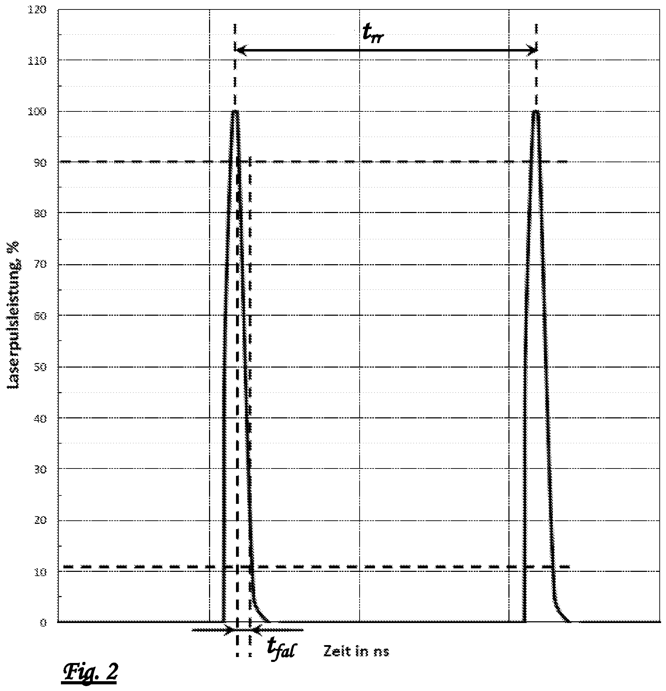

Die eingesetzte erste gepulste Lichtquelle 3 emittiert im Spektralbereich der Anregungsstrahlung für die Moleküle des Sensorfarbstoffes, zum Beispiel 405 nm oder 488 nm. Fig. 2 zeigt bevorzugte Eigenschaften einer gepulsten Lichtquelle, wie sie z.B. in der ersten Sensorvorrichtung 1 eingesetzt werden kann. Der Spektralbereich der Anregungsstrahlung wird durch die optischen Eigenschaften des Sensorfarbstoffes selektiven Plätze bestimmt. Die Abklingzeit $t_{fal}$ der gepulsten Lichtquelle sollte 0,1 ns oder kürzer sein. Die Wiederholungsrate der gepulsten Lichtquelle sollte bevorzugt im Bereich von Megahertz (MHz) oder Kilohertz (kHz) sein. Bei einer Zeitdauer $t_{rr}$ zwischen zwei Pulsen der Lichtquelle 3 von 20 ns beträgt die Wiederholungsrate $1/t_{rr}$ der gepulsten Lichtquelle 50 MHz. Eine Wiederholungsrate von zum Beispiel 50 MHz erlaubt es, Lumineszenz-Abklingzeit bis zu 20 ns zu messen.

Der Photonenzähler 4 detektiert die ankommenden Photonen in Abhängigkeit von der Zeit. Die Zeitauflösung des Photonenzähler 4 sollte im Bereich von 100 ps (0,1 ns) oder besser liegen. Um das Verhältnis des Signals zum Rauschen zu erhöhen und Messrauschen zu eliminieren, kann die Messung über mehrere Pulse der Lichtquelle 3 durchgeführt werden.

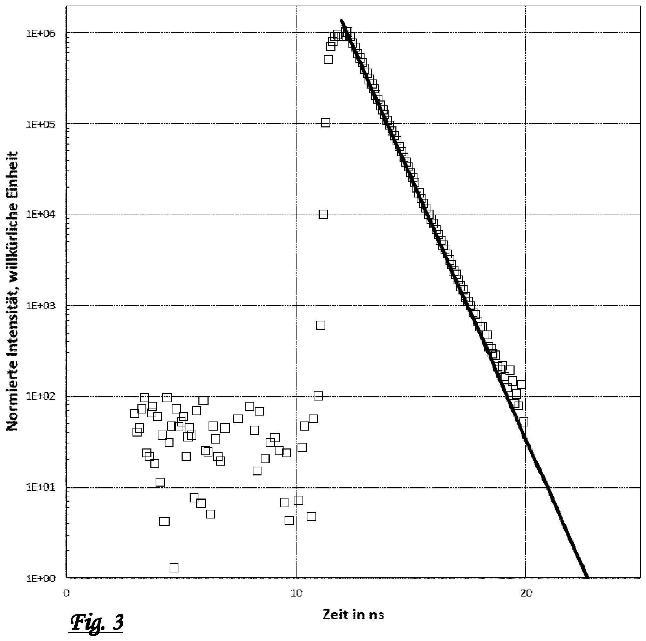

Fig. 3 zeigt das vom Photonenzähler 4 nach Verstärkung und Umwandlung in digitale Form generierte elektrische Signal. Die einzelnen Messpunkte stellen jeweils die zu einem bestimmten Zeitpunkt vom Photonenzähler festgestellte Strahlungsintensität dar. Beispielhaft wurden mit der ersten gepulsten Lichtquelle 3 Laserpulse mit einer Wellenlänge von 488 nm erzeugt, die zu einer zeitabhängigen Fluoreszenz-Antwort führt.

Der an die Messpunkte angepasste Graph in Fig. 3 ist in der logarithmischen Darstellung des Diagramms eine Gerade und zeigt das zeitaufgelöste Fluoreszenz-Abklingen, wobei das Sensorelement 5 der ersten Sensorvorrichtung 1 für Natriumionen ($Na^+$) als Analyt selektiv ist. Die Messung wurde mit einer bekannten Natriumkonzentration von 15 mmol pro Liter durchgeführt. Der Graph wurde auf eine Million Zählimpulse normiert und der Bereich der Abklingphase an eine exponentielle Abklingfunktion angepasst.

Für den Graphen ist im Bereich der Abklingphase die normierte Intensität

$$I = I_{max} \exp\left( -\frac{t - t_{delay}}{\tau} \right)$$

mit $I_{max}$ = 1$E$6, $t_{delay}$ = 12,2 *ns*, und $\tau$ = 0,75 ns,
wobei t die Zeit, $\tau$ die experimentell ermittelte Lumineszenz-Lebensdauer und $t_{delay}$ die von der Länge des Signalübertragungsweges abhängige zeitliche Verzögerung zwischen dem Auslösesignal der Stromversorgung für die Lichtquelle 3 und dem Signal des Photonenzählers 4 ist. Aus dem an die Messpunkte angepassten Graphen kann somit bei bekanntem $t_{delay}$ die Luminszenz-Lebensdauer ermittelt werden.

Anhand der Proportionalität

$$\tau \approx \tau^* \exp\left( \frac{R}{r - r_o} \right)$$

und den bekannten Größen R und $r_0$ lässt sich der mittlere Abstand r der Partikel des Analyten in der Probenflüssigkeit und damit die gesuchte Analyt-Konzentration ermitteln.

$\tau$ entspricht der Lumineszenz-Lebensdauer, die aus dem gemessenen Abklingen der Lumineszenz gemäß der Formel

$$I = I_{max} \exp\left( -\frac{t - t_{delay}}{\tau} \right)$$

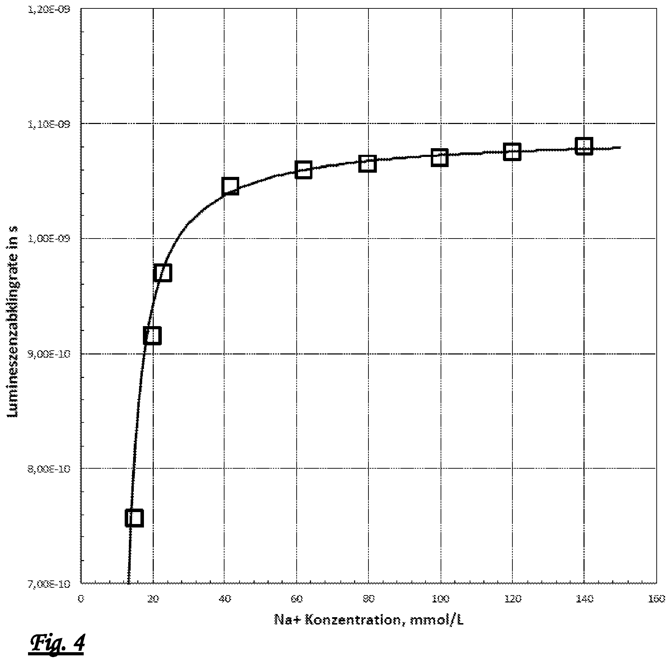

bestimmt wird. R ist der mittlere Abstand zwischen den immobilisierten Molekülen des Sensorfarbstoffes und ist durch das bei der Herstellung der Optode vorgesehene Design der Trägereinheit, z.B. eine Membran, festgelegt. $r_0$ ist der theoretisch berechenbare oder experimentell bestimmbare maximale Abstand zwischen einem Partikel des Analyten und jedem beteiligten Molekül des Sensorfarbstoffes, innerhalb dessen eine Interaktion zwischen dem Partikel des Analyten und den Molekülen des Sensorfarbstoffes stattfinden kann und eine indirekte Austauschwechselwirkung zwischen den beteiligten Sensorfarbstoff-Molekülen auftritt (Förster-Radius). $\tau^*$ ist die durch die indirekte Austauschwechselwirkung erzeugte Zunahme der Lebensdauer der Lumineszenz des Sensorfarbstoffes. Auch dieser Parameter kann für jeden Sensorfarbstoff theoretisch berechnet oder experimentell bestimmt werden (Zwischenmolekulare Energiewanderung und Fluoreszenz, Annalen der Physik, Volume 437, Ausgabe 1-2, [1948], S. 55-75). Für die in Fig. 4 gezeigte Abhängigkeit ergibt sich ein Wert von $\tau^*$ = 0.6 ns.

Somit lässt sich ohne Referenzmessung die gesuchte Konzentration bestimmen.

Fig. 4 zeigt die Abhängigkeit der Konzentration von Natrium-Ionen in der untersuchten Probenflüssigkeit von der Abklingrate der Lumineszenz. Die Abklingrate ist als ein Äquivalent zum Begriff der Lebensdauer zu verstehen. Anhand dieses Graphen lässt sich bei einer gegebener Sensorvorrichtung nach einer Mehrzahl durchgeführter Messungen bei weiteren Messungen aus der Lumineszenz-Lebensdauer unmittelbar die Konzentration der Natrium-Ionen ermitteln.

Fig. 1b) zeigt eine zweite Sensorvorrichtung 9, bei der eine Mehrzahl von Lichtstrahlen, von einer gepulsten Lichtquelle 10 generiert, über einen dichroitischen Spiegel 11 und anschließend über jeweils einen Kern einer Mehrfachkern-Lichtleitfaser 12 zu einem in einem Probenbehälter 15 angeordneten zweiten Sensorelement 13, welches mehrere Sensor-Teilelemente aufweist, gelangt. Jedes Sensor-Teilelement kann für einen bestimmten Analyten vorgesehen sein, so dass in einer Abfolge von mehreren Messungen die Konzentrationen mehrerer Analyten bestimmt werden können.

Die vom Sensorelement 13 ausgehende Lumineszenz-Strahlung wird über die Mehrfachkern-Lichtleitfaser 12 und den dichroitischen Spiegel 11 auf einen Photonenzähler 14 geführt. Der dichroitische Spiegel 11 reflektiert zum einen die Strahlung der Lichtquelle, z.B. Laserlicht der Wellenlänge 400 bis 500 nm, in Richtung auf die Probe und lässt zum anderen die vom Sensorelement 13 ausgehende Lumineszenz-Strahlung zum Photonenzähler 14 hin passieren.

Eine dritte Sensorvorrichtung 16 gemäß Fig. 1c) weist mit einer gepulsten Lichtquelle 17, einem dichroitischen Spiegel 18, einem Probenbehälter 19 und einem aus Sensor-Teilelementen bestehenden Sensorelement 20 einen ähnlichen Aufbau wie die zweite Sensorvorrichtung 9 gemäß Fig. 1.b) auf. Auf die Mehrfachkern-Lichtleitfaser 12 der zweiten Sensorvorrichtung 9 wird allerdings verzichtet. Hier ist vielmehr eine freie Strahlführung zum Sensorelement 20 hin und von dort zu einem Photonenzähler 21 vorgesehen.

In der Variante einer vierten Sensorvorrichtung 22 leitet eine erste Lichtleitfaser 23 die von einer gepulsten Lichtquelle 25 generierte Strahlung zu einem in einem Probenbehälter 27 angeordneten vierten Sensorelement 26 und eine zweite Lichtleitfaser 24 Lumineszenz-Strahlung vom vierten Sensorelement 26 in Richtung auf einen Photonenzähler 28. Vor Auftreffen auf den vierten Photonenzähler 28 durchläuft die Lumineszenz-Strahlung einen optischen Filter 29.

Im Folgenden wird als Alternative zum zeitauflösenden Verfahren ein Verfahren unter Ausnutzung der Lichtpolarisation dargestellt.

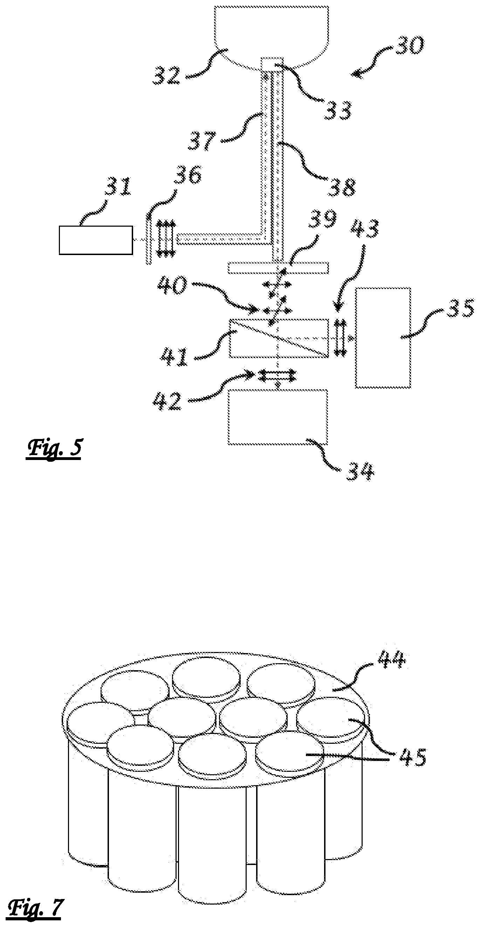

Fig. 5 zeigt eine fünfte Sensorvorrichtung 30 (Optode) mit einer nicht gepulsten, sondern kontinuierlichen Lichtquelle 31 (z.B. Laser oder LED), einem in einem Probenbehälter 32 angeordneten Sensorelement 33, einem ersten Photodetektor 34 und einem zweiten Photodetektor 35. Nach Verlassen der Lichtquelle 38 durchläuft die Strahlung einen Linearpolarisator 36 zur Erzeugung einer linearen Polarisation der Strahlung. Anschließend führt eine erste Lichtleitfaser 37 das linear polarisierte Anregungslicht 40 bis zum Sensorelement 33, wo es zur optischen Anregung dient. Im Sensorelement 33 kommt es zu einer von der Konzentration des Analyten abhängigen Lumineszenz-Antwort auf die linear polarisierte optische Anregung.

Ein aufgefangener Teil der Lumineszenz-Strahlung wird über eine zweite Lichtleitfaser 38 in Richtung auf einen optischen Filter 39, z.B. einen Hochpass-Filter, geführt, der insbesondere gestreutes Anregungslicht ausfiltert. Mittels eines polarisierenden Strahlteilers 41 wird die auftreffende Lumineszenzstrahlung in zwei Teilstrahlen 42 und 43 aufgespalten, die senkrecht aufeinander stehende Polarisationen aufweisen. Die Polarisationsrichtungen sind jeweils durch Doppelpfeile symbolisiert. Der erste Teilstrahl 42 fällt auf den ersten Photodetektor 34, der zweite Teilstrahl 43 auf den zweiten Photodetektor 35. Mittels der Photodetektoren 34 und 35 wird der Polarisationsgrad

$$P = \left| \frac{I_{II} - I_{\perp}}{I_{II} + I_{\perp}} \right|$$

der Lumineszenzstrahlung ermittelt, wobei $I_{\parallel}$ und $I_{\perp}$ die Intensitäten der Teilstrahlen mit senkrecht zueinander stehenden Polarisationsrichtungen darstellen. Der Polarisationsgrad weist die Proportionalität

$$P \sim \exp\left( \frac{R}{r - r_o} \right)$$

auf, weshalb hieraus über die bekannten Größen R und $r_0$ sowie die Beziehung zwischen r und der gesuchten Analytkonzentration (siehe Erläuterungen in der Beschreibungseinleitung) letztere ermittelt werden kann.

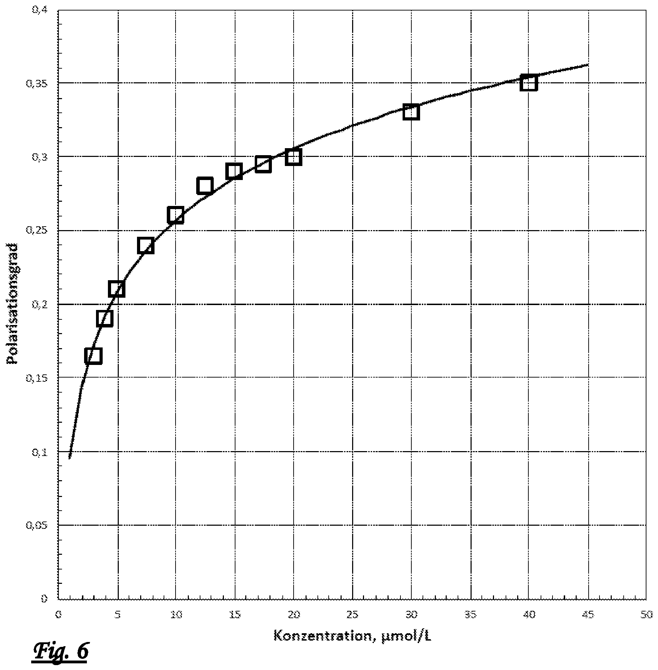

Fig. 6 zeigt graphisch eine derart ermittelte Abhängigkeit des Polarisationsgrades der Lumineszenz-Strahlung von der Konzentration des Analyten.

Fig. 7 zeigt beispielhaft eine Multikern-Lichtleitfaser 44 mit zehn Kernsträngen 45, wobei jeder der Stränge 45 wie eine eigenständige Lichtleitfaser wirkt und in jeweils einem eigenen Sensorelement endet.

**Bezugszeichenliste**

| | | | |
|---|---|---|---|
| 1 | erste Sensorvorrichtung | 31 | kontinuierliche Lichtquelle |

(fortgesetzt)

| | | | |
|---|---|---|---|
| 2 | ersten Probenbehälter | 32 | Probenbehälter |
| 3 | erste gepulste Lichtquelle | 33 | Sensorelement |
| 4 | Photonenzähler | 34 | erster Photodetektor |
| 5 | Sensorelement | 35 | zweiter Photodetektor |
| 6 | Strahlteiler | 36 | Linearpolarisator |
| 7 | Lichtleitfaser | 37 | erste Lichtleitfaser |
| 8 | erster optischer Filter | 38 | zweite Lichtleitfaser |
| 9 | zweite Sensorvorrichtung | 39 | optischer Filter |
| 10 | zweite gepulste Lichtquelle | 40 | linear polarisiertes Anregungslicht |
| 12 | Mehrfachkern-Lichtleitfaser | 41 | polarisierender Strahlteiler |
| 13 | zweites Sensorelement | 42 | erster Teilstrahl |
| 14 | Photonenzähler | 43 | zweiter Teilstrahl |
| 15 | Probenbehälter | 44 | Multikern-Lichtleitfaser |
| 16 | dritte Sensorvorrichtung | 45 | Kernstrang |
| 17 | dritte gepulste Lichtquelle | | |
| 18 | dichroitischer Spiegel | | |
| 19 | Probenbehälter | | |
| 20 | Sensorelement | | |
| 21 | dritter Photonenzähler | | |
| 22 | vierte Sensorvorrichtung | | |
| 23 | Lichtleitfaser | | |
| 24 | Lichtleitfaser | | |
| 25 | vierte gepulste Lichtquelle | | |
| 26 | viertes Sensorelement | | |
| 27 | Probenbehälter | | |
| 28 | vierter Photonenzähler | | |
| 29 | optischer Filter | | |
| 30 | fünfte Sensorvorrichtung | | |

## Patentansprüche

1. Verfahren zur Bestimmung der Konzentration eines Analyten in einer Probenflüssigkeit, bei dem

a) mittels einer Strahlungsquelle (3, 10, 17, 25, 31) Anregungsstrahlung auf eine mit der Probenflüssigkeit in Kontakt stehende, immobilisierte Moleküle eines für den Analyten sensitiven Sensorfarbstoffes aufweisende Trägereinheit (5, 13, 20, 26, 33) gerichtet wird, wobei der mittlere Abstand R zwischen immobilisierten Molekülen kleiner als der Förster-Radius $r_0$ ist,
b) ein Anteil einer durch die Anregungsstrahlung ausgelösten Lumineszenzstrahlung des Sensorfarbstoffes auf einen Strahlungsdetektor (4, 14, 21, 28, 34, 35) gegeben wird, um daraus ein Detektorausgangssignal zu generieren, und
c) eine Auswerteroutine die Analytkonzentration aus dem Detektorausgangssignal ermittelt, wobei eine Abhängigkeit einer Eigenschaft der Lumineszenzstrahlung von der Konzentration des Analyten in der Probenflüssigkeit ausgenutzt wird,
**dadurch gekennzeichnet, dass**
d) für die Ermittlung der Analytkonzentration die Abhängigkeit der untersuchten Eigenschaft der Lumineszenzstrahlung von der indirekten Austauschwechselwirkung zwischen den Molekülen des Sensorfarbstoffes untereinander ausgenutzt wird, wobei die indirekte Austauschwechselwirkung stattfindet, wenn die Moleküle des Sensorfarbstoffes mit den Partikeln des Analyten interagieren und
e) die Auswerteroutine eine zur Exponentialfunktion

$$\exp\left(\frac{R}{r - r_o}\right)$$

proportionale Größe auswertet, wobei

R ein Maß für den Abstand zwischen den immobilisierten Molekülen des Sensorfarbstoffes ist,
r ein Maß für den Abstand zwischen den Molekülen des Analyten ist, die mit den Molekülen des Sensorfarbstoffes interagieren und
$r_0$ der Förster-Radius ist.

2. Verfahren nach Anspruch 1, **dadurch gekennzeichnet, dass** als Eigenschaft der Lumineszenzstrahlung die Abhängigkeit der Lebensdauer der Lumineszenzstrahlung von der Analytkonzentration ausgenutzt wird und die Auswerteroutine zur Ermittlung der Analytkonzentration von einer bekannten Konzentration der in der Polymermatrix immobilisierten Sensorfarbstoff-Moleküle und einer bekannten Abhängigkeit der Lebensdauer der Lumineszenzstrahlung von der Sensorfarbstoff-Molekülkonzentration sowie der Analytkonzentration ausgeht.

3. Verfahren nach Anspruch 2, **dadurch gekennzeichnet, dass** für die Abhängigkeit der Lebensdauer der Lumineszenzstrahlung von der Sensorfarbstoff-Molekülkonzentration sowie der Analyt-Konzentration die Proportionalität

$$\tau \sim \exp\left(\frac{R}{r - r_o}\right)$$

herangezogen wird, wobei
$\tau$ die Lebensdauer der Lumineszenzstrahlung der immobilisierten und mit dem Analyten wechselwirkenden Moleküle des Sensorfarbstoffes ist.

4. Verfahren nach Anspruch 2 oder 3, **dadurch gekennzeichnet, dass** die Lebensdauer der Lumineszenzstrahlung mittels zeitaufgelöster Messung ermittelt wird.

5. Verfahren nach Anspruch 2 oder 3, **dadurch gekennzeichnet, dass** die Lebensdauer der Lumineszenzstrahlung mittels Phasenmodulation ermittelt wird.

6. Verfahren nach Anspruch 1, **dadurch gekennzeichnet, dass**

a) die Anregungsstrahlung polarisiert auf die Trägereinheit gerichtet wird und
b) für zwei im Wesentlichen senkrecht aufeinander stehende Polarisationsrichtungen gegebene Intensitäten $I_{\parallel}$ und $I_{\perp}$ einer durch die Anregungsstrahlung ausgelösten Lumineszenzstrahlung des Sensorfarbstoffes festgestellt werden und als Eigenschaft der Lumineszenzstrahlung die Abhängigkeit des Polarisationsgrades

$$P = \left|\frac{I_{II} - I_{\perp}}{I_{II} + I_{\perp}}\right|$$

von der Konzentration des Analyten genutzt wird.

7. Verfahren nach Anspruch 6, **dadurch gekennzeichnet, dass** die Funktion

$$P \sim \exp\left(\frac{R}{r - r_o}\right),$$

genutzt wird.

**8.** Optode zur Bestimmung der Konzentration eines Analyten in einer Probenflüssigkeit, umfassend

a) eine immobilisierte Moleküle eines Sensorfarbstoffes aufweisende Trägereinheit (5, 13, 20, 26, 33), wobei der mittlere Abstand R zwischen immobilisierten Molekülen kleiner als der Förster-Radius $r_0$ ist,
b) eine Strahlungsquelle (3, 10, 17, 25, 31) für eine Anregungsstrahlung,
c) einen Strahlungsdetektor (4, 14, 21, 28, 34, 35) und
d) eine Auswerteeinheit mit einer Auswerteroutine, um aus der Lebensdauer einer durch die Anregungsstrahlung ausgelösten Lumineszenzstrahlung der immobilisierten Sensorfarbstoff-Moleküle die Konzentration des Analyten zu ermitteln,
**dadurch gekennzeichnet, dass**
e) die Optode eingerichtet ist, das Verfahren nach einem der Ansprüche 1 bis 7 durchzuführen.


**Claims**

**1.** Method for determining a concentration of an analyte in a sample liquid, in which,

a) by means of a radiation source (3, 10, 17, 25, 31), excitation radiation is directed onto a carrier unit (5, 13, 20, 26, 33) which is in contact with the sample liquid and which has immobilized molecules of a sensor dye that is sensitive to the analyte, wherein the mean distance R between immobilized molecules is smaller than the Förster radius $r_0$,
b) a portion of sensor dye luminescence radiation, the sensor dye luminescence radiation being induced by the excitation radiation, is applied to a radiation detector (4, 14, 21, 28, 34, 35) in order to generate a detector output signal therefrom, and
c) an evaluation routine determines the analyte concentration from the detector output signal, wherein a dependence of a property of the luminescence radiation on the concentration of the analyte in the sample liquid is used,
**characterized in that**
d) for determining the analyte concentration, the dependency of the property of the luminescence radiation being examined on an indirect exchange interaction between the molecules of the sensor dye with one another is exploited, wherein the indirect exchange interaction occurs when the molecules of the sensor dye interact with the particles of the analyte and
e) that the evaluation routine evaluates a variable that is proportional to an exponential function

$$\exp\left(\frac{R}{r - r_o}\right)$$

wherein R is a measure of a distance between the immobilized molecules of the sensor dye,
r is a measure of the distance between the molecules of the analyte that interact with a molecule of the sensor dye, and
$r_0$ is the Förster radius.

**2.** Method according to claim 1, **characterized in that** the dependence of a luminescence radiation lifetime on the analyte concentration is used as the property of the luminescence radiation and the evaluation routine for ascertaining the analyte concentration is based on a known concentration of the sensor dye molecules immobilized in the polymer matrix and a known dependence of the lifetime of the luminescence radiation on the sensor dye molecule concentration and the analyte concentration.

**3.** Method according to claim 2, **characterized in that**, for the dependence of the lifetime of the luminescence radiation on the sensor dye molecule concentration and the analyte concentration, the proportionality

$$\tau \sim \exp\left(\frac{R}{r - r_o}\right)$$

is used, wherein

$\tau$ is the lifetime of the luminescence radiation of the immobilized molecules of the sensor dye which interact with the particles of the analyte.

4. Method according to claim 2 or 3, **characterized in that** the lifetime of the luminescence radiation is ascertained by means of time-resolved measurement.

5. Method according to claim 2 or 3, **characterized in that** the lifetime of the luminescence radiation is ascertained by means of phase modulation.

6. Method according to claim 1, **characterized in that**

    a) the excitation radiation is directed onto the carrier unit in a polarized manner and
    b) given intensities $I_{\parallel}$ and $I\perp$ of a sensor dye luminescence radiation, induced by the excitation radiation, are determined for two essentially mutually perpendicular polarization directions and, as the property of the luminescence radiation the dependence of the degree of polarization

$$P = \left| \frac{I_{II} - I_{\perp}}{I_{II} + I_{\perp}} \right|$$

on the concentration of the analyte is used.

7. Method according to claim 6, **characterized in that** the function

$$P \sim \exp\left( \frac{R}{r - r_o} \right)$$

is used.

8. Optode for determining the concentration of an analyte in a sample liquid, comprising

    a) a carrier unit (5, 13, 20, 26, 33) having immobilized molecules of a sensor dye, wherein the average distance R between immobilized molecules is smaller than the Förster radius $r_0$.
    b) a radiation source (3, 10, 17, 25, 31) for excitation radiation,
    c) a radiation detector (4, 14, 21, 28, 34, 35), and
    d) an evaluation unit that has an evaluation routine in order to ascertain the concentration of the analyte from the lifetime of luminescence radiation of the immobilized sensor dye molecules, which radiation is induced by the excitation radiation,
    **characterized in that**
    e) the optode is designed to carry out the method according to one of claims 1 to 7.

**Revendications**

1. Procédé de détermination de la concentration d'un analyte dans un liquide échantillon, procédé dans lequel

    a) au moyen d'une source de rayonnement (3, 10, 17, 25, 31), un rayonnement d'excitation est dirigé sur une unité porteuse (5, 13, 20, 26, 33) qui est en contact avec le liquide échantillon et qui comporte des molécules immobilisées d'un colorant de détection sensible à l'analyte,
    b) une partie d'un rayonnement luminescent du colorant de détection déclenché par le rayonnement d'excitation est appliquée à un détecteur de rayonnement (4, 14, 21, 28, 34, 35) afin de générer à partir de là un signal de sortie de détecteur, et
    c) une routine d'évaluation détermine la concentration d'analyte à partir du signal de sortie du détecteur, une relation de dépendance étant utilisée entre une propriété du rayonnement luminescent et la concentration de l'analyte dans le liquide échantillon,
**caractérisé en ce que**
d) la relation de dépendance entre la propriété étudiée du rayonnement luminescent et une interaction d'échange indirecte entre les molécules du colorant de détection étant utilisée pour déterminer la concentration d'analyte, l'interaction d'échange indirecte ayant lieu lorsque les molécules du colorant de détection interagissent avec les particules de l'analyte,
e) la routine d'évaluation évalue une quantité proportionnelle à la fonction exponentielle

$$\exp\left(\frac{R}{r - r_o}\right),$$

R étant une mesure de la distance entre les molécules immobilisées du colorant de détection,
r étant une mesure de la distance entre les molécules de l'analyte qui interagissent avec les molécules du colorant de détection et
$r_0$ étant le rayon de Förster.

2. Procédé selon la revendication 1, **caractérisé en ce que** la relation de dépendance entre la durée de vie du rayonnement luminescent et la concentration d'analyte est utilisée comme propriété du rayonnement luminescent et la routine d'évaluation pour déterminer la concentration d'analyte est basée sur une concentration connue des molécules de colorant de détection immobilisées dans la matrice polymère et une relation de dépendance connue entre la durée de vie du rayonnement luminescent et la concentration de molécules de colorant de détection ainsi que la concentration d'analyte.

3. Procédé selon la revendication 2, **caractérisé en ce que** la proportionnalité

$$\tau \sim \exp\left(\frac{R}{r - r_o}\right)$$

est utilisée pour la relation de dépendance entre la durée de vie du rayonnement luminescent et la concentration de molécules de colorant de détection ainsi que la concentration d'analyte,
$\tau$ étant la durée de vie du rayonnement luminescent des molécules immobilisées du colorant de détection et interagissant avec l'analyte.

4. Procédé selon la revendication 2 ou 3, **caractérisé en ce que** la durée de vie du rayonnement luminescent est déterminée au moyen d'une mesure à résolution temporelle.

5. Procédé selon la revendication 2 ou 3, **caractérisé en ce que** la durée de vie du rayonnement luminescent est déterminée au moyen d'une modulation de phase.

6. Procédé selon la revendication 1, **caractérisé en ce que**

a) le rayonnement d'excitation est polarisé et dirigé sur l'unité porteuse et
b) des intensités données $I_{\parallel}$ et $I\perp$ d'un rayonnement luminescent du colorant de détection, déclenché par le rayonnement d'excitation, sont définies pour deux directions de polarisation sensiblement perpendiculaires et la relation de dépendance entre le degré de polarisation

$$P = \left|\frac{I_{II} - I_{\perp}}{I_{II} + I_{\perp}}\right|$$

et la concentration de l'analyte est utilisée comme propriété du rayonnement luminescent.

7. Procédé selon la revendication 6, **caractérisé en ce que** la fonction

$$P \sim \exp\left(\frac{R}{r - r_0}\right)$$

est utilisée.

**8.** Optode destinée à la détermination de la concentration d'un analyte dans un liquide échantillon, ladite optode comprenant

  a) une unité porteuse (5, 13, 20, 26, 33) qui comporte des molécules immobilisées d'un colorant de détection, la distance moyenne entre les molécules immobilisées étant inférieure au rayon de Förster,
  b) une source de rayonnement (3, 10, 17, 25, 31) destinée à un rayonnement d'excitation,
  c) un détecteur de rayonnement (4, 14, 21, 28, 34, 35) et
  d) une unité d'évaluation qui comprend une routine d'évaluation afin de déterminer la concentration de l'analyte à partir de la durée de vie d'un rayonnement luminescent des molécules de colorant de détection immobilisées qui est déclenché par le rayonnement d'excitation, **caractérisée en ce que**
  e) le dispositif est conçu pour mettre en oeuvre le procédé selon l'une des revendications 1 à 7.

_Fig. 1_

_Fig. 2_

*Fig. 3*

*Fig. 4*

**Fig. 6**

_Fig. 5_

_Fig. 7_

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2805151 B1
- US 5246867 A
- US 6395556 B1
- US 20100105048 A1
- US 20060014175 A1
- DE 102018204744 A1
- US 2009246888 A1

**In der Beschreibung aufgeführte Nicht-Patentliteratur**

- **THEODOR FÖRSTER.** Experimentelle und theoretische Untersuchung des zwischenmolekularen Übergangs von Elektronenanregungsenergie. *Zeitschrift für Naturforschung,* 1949, vol. 4a, 321-327
- Zwischenmolekulare Energiewanderung und Fluoreszenz. **T. FÖRSTER.** Annalen der Physik. 1948, vol. 437, 55-75
- Zwischenmolekulare Energiewanderung und Fluoreszenz. Annalen der Physik. 1948, vol. 437, 55-75